(19) Europäisches Patentamt
European Patent Office
Office européen des brevets

(11) **EP 2 153 845 A1**

(12) **EUROPEAN PATENT APPLICATION**

(43) Date of publication:
**17.02.2010 Bulletin 2010/07**

(51) Int Cl.:
*A61K 39/00* (2006.01)    *C07K 16/28* (2006.01)
*A61P 35/00* (2006.01)

(21) Application number: **08014455.3**

(22) Date of filing: **13.08.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Julius-Maximilians-Universität Würzburg**
**97070 Würzburg (DE)**

(72) Inventors:
• **Becker, Jürgen**
**97082 Würzburg (DE)**
• **Hofmeister, Valeska**
**97082 Würzburg (DE)**
• **Schrama, David**
**97204 Höchberg (DE)**

(74) Representative: **Elbel, Michaela**
**Rothkopf Theobald Elbel**
**Patent- und Rechtsanwälte**
**Altheimer Eck 13**
**80331 München (DE)**

(54) **Endoglin peptides, vaccines and methods for preparing the same**

(57)   The present invention concerns artificial peptides derived from endoglin (CD105) for the treatment of cancer. Furthermore, the invention relates to polypeptides comprising the artificial peptide, nucleic acids encoding the artificial peptide or polypeptide, antibodies against the artificial peptide and vaccines comprising at least one substance selected from the group consisting of a peptide, a nucleic acid and an antibody for the treatment of cancer. The present invention also concerns a method for preparing a vaccine and the use of a peptide, polypeptide, nucleic acid or antibody for the manufacture of a drug and/or a vaccine for the treatment and/or prevention of cancer or an immunological disorder.

Fig. 4

**Description**

**Field of the invention**

The present invention concerns the technical field of artificial peptides. Especially, the invention relates to polypeptides derived from endoglin (CD105) for the treatment of cancer, polypeptides comprising the artificial peptide, nucleic acids encoding the artificial peptide or polypeptide, antibodies against the artificial peptide and vaccines for the treatment of cancer. The present invention also concerns a method for preparing a vaccine and the use of a peptide, polypeptide, nucleic acid or antibody according to the invention for the manufacture of a drug and/or a vaccine for the treatment and/or prevention of cancer or an immunological disorder.

**Background of the invention**

It is estimated that in the U.S. more than 1,400,000 and in Europe about 3,200,000 new cancer cases would be diagnosed each year, and more than half a million people in the U.S. and about 1,700,000 people in Europe would die from cancer each year. While early and localized diseases may effectively be treated by radical excision metastatic cancer is fatal in most cases. Nevertheless, some patients show spontaneous regression of both primary tumors and metastases. This event is largely attributed to adaptive immune responses, and the presence of cytotoxic T cells infiltrating the tumor is associated with a better clinical prognosis (Kondratiev et al., 2004). In addition, the increased tumor incidence in immune suppressed individuals indicates that cancer, at least in part, can be controlled by the immune system (Adami et al., 2003). Therefore, efforts are being made to stimulate patient's immune effector cells to recognize and destroy cancer cells.

The majority of current vaccination strategies is based on tumor-associated antigens (TAAs). But clinical efficacy of these immunotherapies is limited, because neoplastic cells may be subject of several immune evasion mechanisms, e.g. downregulation of antigen expression or presentation of the respective antigen. The presentation of TAA-derived peptides to cytotoxic T cells can be lost by mutation, deletion or silencing of the TAA gene itself or by defects in the antigen presentation pathway, e.g. loss of major histocompatibility complex class I heavy chains, $\beta$2-microglobulin, TAP or tapasin expression. Therefore, antigens contributing to survival of tumor cells seem to be more suitable therapeutic targets than neoplastic antigens themselves. However, even such contributing antigens may be subject to immune evasion due to impaired processing and cell surface expression.

Carcinogenesis is a process depending on genetic and epigenetic alterations accumulated in transforming cells. Nevertheless, many steps necessary for tumor progression, e.g. proliferation, invasion, angiogenesis, and metastasis are influenced by microenvironmental factors, such as growth factors, angiogenic factors, cytokines, and proteolytic enzymes. During transformation theses factors are provided by normal cells, i.e. fibroblasts, endothelial, and immunocompetent cells due to reciprocal interactions between neoplastic and adjacent normal cells. Indeed, the microenvironment influences the stromal cells in such a way that they rather promote than inhibit tumor progression by allowing vasculogenesis and angiogenesis, recruitment of reactive stromal fibroblasts, lymphoid and phagocytic infiltrates, secretion of peptide mediators, and proteolytic enzymes, as well as production of a modified extracellular matrix. Tumor stromal cells may serve as targets for immune intervention, and several antigens have been identified, which are induced or upregulated on the tumor stroma.

Endothelial cells are possible targets for immunotherapy. Advantageously, they are highly accessible to antibodies or lytic effector cells. The immunological destruction of tumor blood vessels should lead to a widespread necrosis in solid tumors, hence inflicting its effect upon tumor cells. On tumor endothelial cells numerous molecules are expressed, like growth factor receptors, such as VEGF receptors, cell surface antigens with enzymatic activity, such as prostate-specific membrane antigens, and functionally uncharacterized antigens, such as tumor endothelial markers. Therefore, there is a wide range of endothelial antigens that may be targets for immunotherapy.

An endothelial marker scrutinized for its role in vascular targeting is CD105, also called endoglin (Hofmeister et al., 2008). CD105 is upregulated on activated endothelium by TGF-$\beta$2 and hypoxia and has been reported to be expressed on tumor endothelial cells of a plethora of solid tumors. Expression of CD105 in these tumors is correlated with vascular density and most importantly, poor prognosis (Dales et al., 2004).

CD105 provides a restricted binding affinity for leukocyte antigen and a restricted capacity to form stable peptide-major histocompatibility complex (MHC) interactions. However, these abilities are critical for immunogenicity and probability to induce production of cytotoxic T cell, which is again responsible for the success of immunotherapies by CD105.

Therefore, there is a need for improved antigens that efficiently induce immune reactions, while undesired side effects and risk of therapy resistance are reduced.

The solution to this problem is achieved by the embodiments of the present invention characterized by the claims, and described further below.

## Summary of the invention

It is an object of the present invention to provide an artificial peptide for the treatment of cancer, the peptide is derived from endoglin and comprises a nonapeptide, which inhibits 50 % of binding of a reference peptide to human leukocyte antigen A2 (HLA-A2) in a competitive binding assay at a concentration below 5 μM of the nonapeptide, wherein the nonapeptide has a leucine or methionine at amino acid position 2 and/or a valine or leucine at amino acid position 9.

Another aspect of the invention is to provide an artificial peptide for the treatment of cancer, the peptide is derived from human endoglin and binds specifically to human leukocyte antigen A2 (HLA-A2), wherein the peptide is selected from the group consisting of SEQ ID NO: 1 to SEQ ID NO: 16.

In addition, the present invention is directed to a polypeptide for the treatment of cancer, the polypeptide comprises an artificial peptide according to the invention.

Furthermore, the invention concerns a nucleic acid for the treatment of cancer, the nucleic acid encodes at least one peptide or polypeptide according to the invention.

Moreover, the invention relates to an antibody against a peptide or a polypeptide according to the invention for the treatment of cancer.

Another object of the present invention is to provide a vaccine comprising at least one substance selected from the group consisting of a peptide, a nucleic acid and an antibody according to the invention.

The invention is also directed to a method for preparing a vaccine comprising the steps of:

a) providing the peptide, the polypeptide or the antibody according to the invention;
b) admixing an adjuvant to the peptide, polypeptide or antibody.

The invention relates also to the use of at least one peptide, polypeptide, nucleic acid or antibody according to the invention for the manufacture of a drug and/or a vaccine for the treatment and/or prevention of cancer or an immunological disorder.

## Brief description of the figures

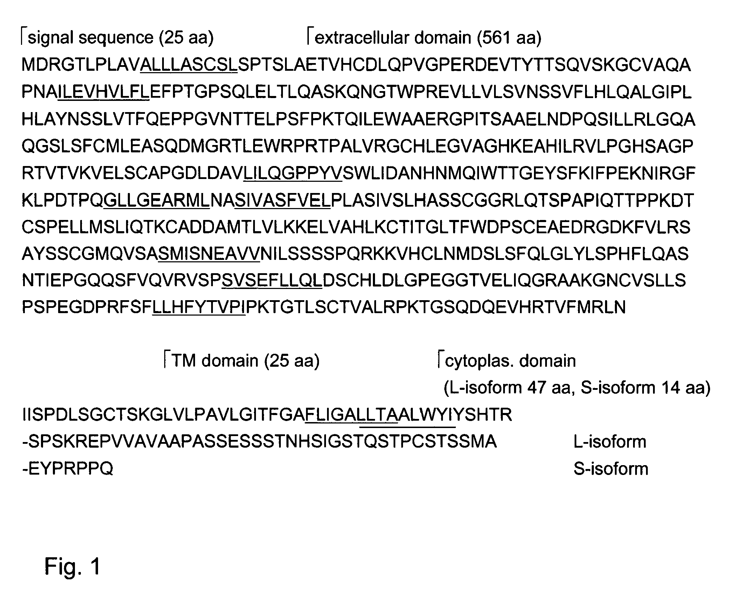

Fig. 1 shows a selection of HLA-A2 restricted CD105 derived peptides. An amino acid sequence of the human CD105 L- and S-isoform is shown in single letter code. The positions of the peptides binding to HLA-A2 are underlined.

Fig. 2 depicts binding of CD105 derived peptides to HLA-A2 tested by a competitive binding assay. $IC_{50}$ value specifies the peptide concentration sufficient for 50% inhibition of binding of a reference peptide.

Fig. 3 shows affinity optimized CD105 derived peptides. Anchor amino acids were exchanged to enhance affinity of nonamer peptides to HLA-A2, wherein leucine or methionine is localized at anchor amino acid position 2 and valine or leucine is localized at anchor amino acid position 9.

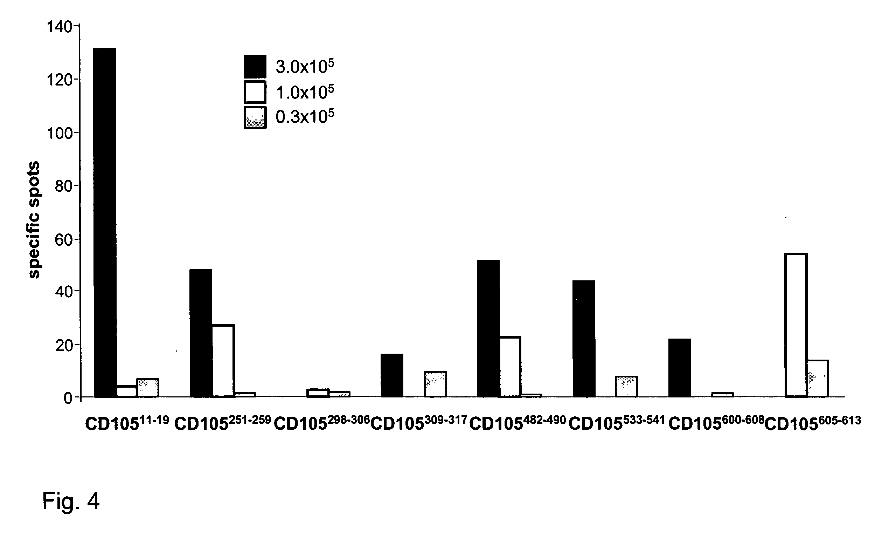

In the diagram of Fig. 4 T cells specific for CD105 derived peptides are detected by IFN-γ ELISPOT. $3 \times 10^5$, $1 \times 10^5$ or $0.3 \times 10^5$ peripheral blood lymphocytes of a melanoma patient were prestimulated *in vitro* by CD105 derived peptides, and reactivities to the respective peptides were analyzed by IFN-γ ELISPOT. Results are shown as specific spots:

$$\text{specific spots} = [\text{mean spots}_{\text{with peptide}} - \text{mean spots}_{\text{without peptide}}].$$

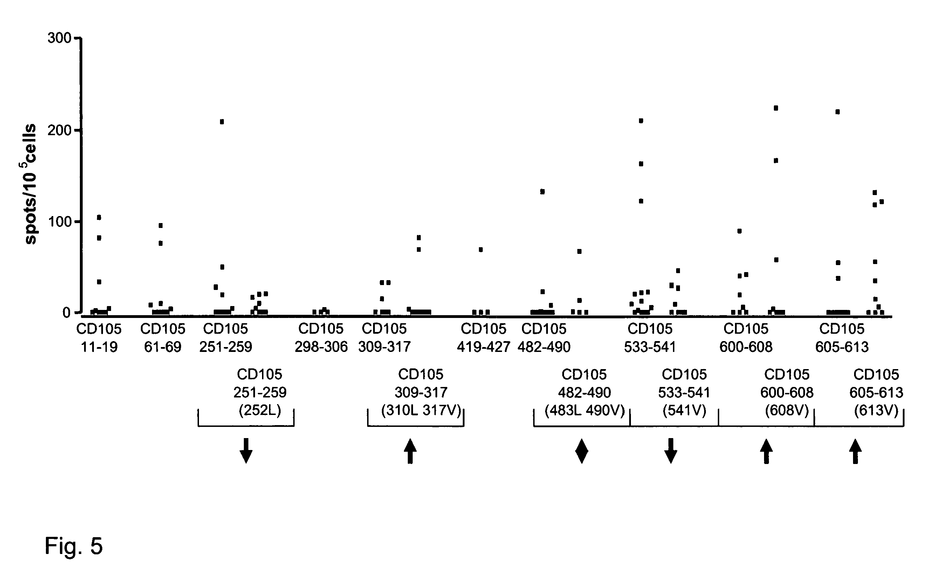

Fig. 5 depicts responses of melanoma patients' peripheral blood lymphocytes to CD105 derived epitopes. IFN-γ responses of peripheral blood lymphocytes of melanoma patients upon stimulation with selected parental and modified CD105 peptides were measured in ELISPOT. An upward arrow means enhanced IFN-γ response, a downward arrow means reduced IFN-γ response, and a rhomb means unchanged IFN-γ response to modified peptides in comparison to parental peptides.

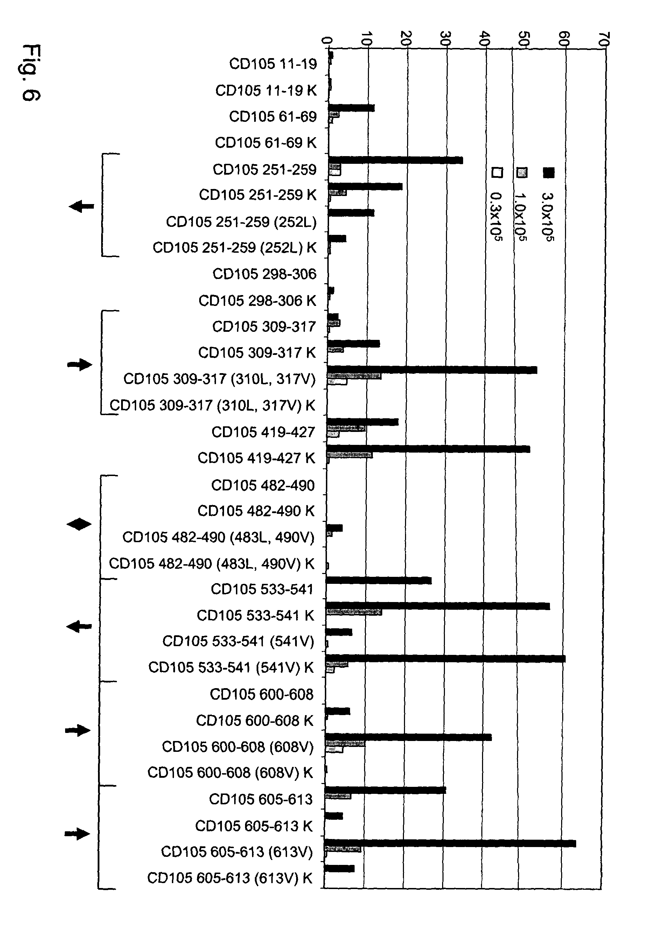

Fig. 6 shows immunogenic effects of CD105 derived peptides in HLA-A2/k$^b$ transgenic mice. Vaccination of transgenic mice with or without (K) parental or modified CD105 peptides was followed by *in vitro* analysis of IFN-γ responses in ELISPOT. The legend is the same as in Fig. 5.

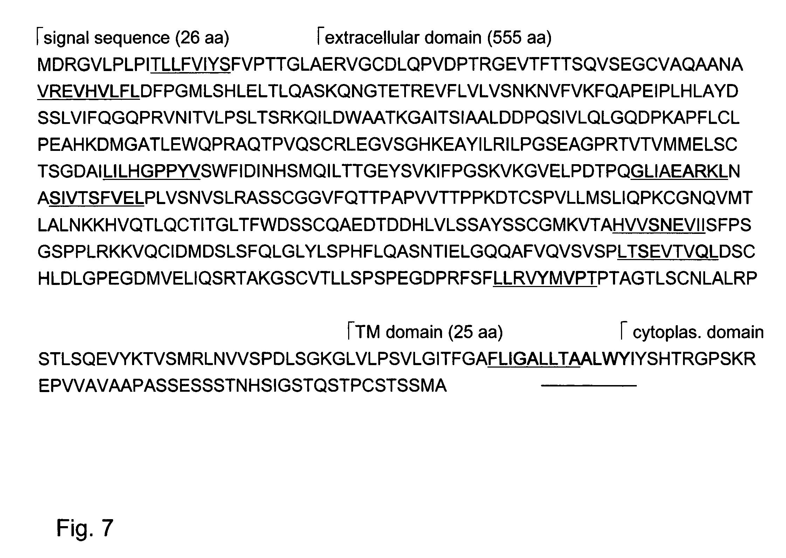

In Fig. 7 murine CD105 is displayed. Amino acid positions that are homologue to human HLA-A2 restricted CD105 peptides are underlined. Amino acids identical to human CD105 peptides are depicted in bold.

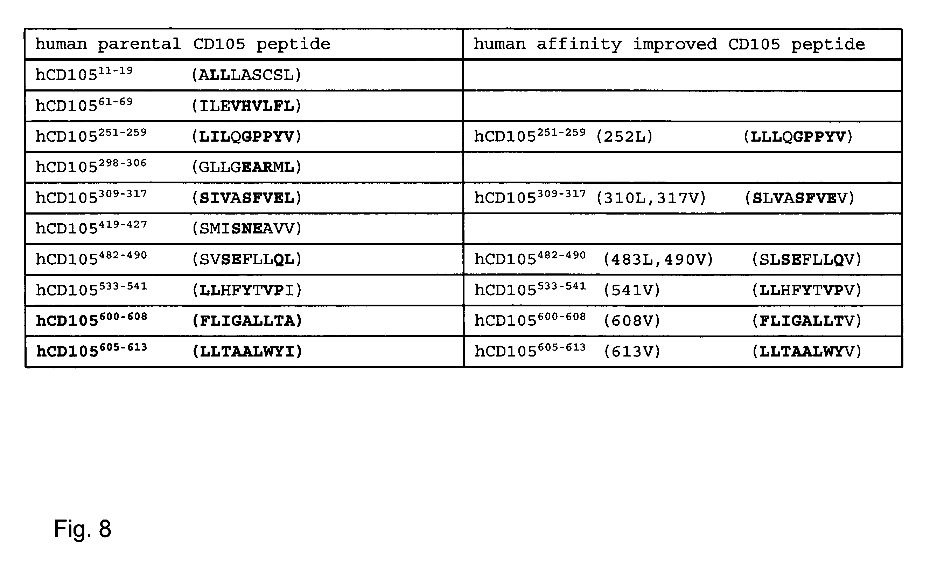

In Fig. 8 HLA-A2 restricted human CD105 derived parental peptides (left column) and affinity improved human CD105 derived peptides (right column) are shown. Amino acids identical to the amino acids in the corresponding position of the parental murine CD105 peptide are depicted in bold.

## Detailed description of the invention

It is an object of the present invention to provide an artificial peptide for the treatment of cancer, the peptide is derived from endoglin comprising a nonapeptide, which inhibits 50 % of binding of a reference peptide to human leukocyte antigen A2 in a competitive binding assay at a concentration below 5 μM of the nonapeptide, wherein the nonapeptide has a leucine or methionine at amino acid position 2 and/or a valine or leucine at amino acid position 9.

The term "peptide" as used herein refers to an x-mer that comprises multiple amino acids linked by peptide bonds regardless of length and conformation. Therefore, the term "peptide" includes chains of several amino acids, oligomers, polymers as well as polypeptides. Non-amino acid units, like steroids or carbohydrates, linked to the polypeptide can also be included.

The term "nonapeptide" as used herein refers to a nonamer including nine amino acids including functional variants, e.g. glycosylation, modification and additionally attached residues.

The term "artificial peptide" as used herein refers to a peptide that is derived from a natural, parental peptide or polypeptide by e.g. modifying, selecting or isolating specific sequences; expression of a mutated nucleic acid; altering amino acid sequence; adding or removing residues; and exchanging, inserting and/or deleting peptide domains, multiple amino acids or single amino acids. Artificial peptides are based on CD105. Preferred modifications are exchanges of at least one sequence of SEQ ID NO: 1 to SEQ ID NO: 10 against SEQ ID NO: 11 to SEQ ID NO: 16. Other preferred modifications are exchanges of single amino acids at position 2 and/or 9 in the context of a nonapeptide.

The term "endoglin" as used herein refers to CD105, which is a 95 kD cell surface polypeptide assembled as a homodimer, which is composed of a signal sequence, an extracellular domain, a transmembrane domain and a cytoplasmic domain. There exists two isoforms of endoglin, L-endoglin and S-endoglin, differing in their C-terminal intracellular domain. CD105 functions as an accessory receptor for kinase receptor complexes of the transforming growth factor (TGF)-β superfamily. Thereby, CD105 modulates TGF-β signaling, i.e. it antagonizes the inhibitory effects of TGF-β1, and CD105 is upregulated on activated endothelium via TGF-β2. In addition, CD105 has an anti-apoptotic effect under hypoxic conditions.

CD105 is expressed as tumor stroma associated antigen. Its expression is not restricted to tumor endothelial cells (TEC). It is present on activated monocytes, differentiated macrophages, early B cells, erythroid precursor cells, fibroblasts, follicular dendritic cells, melanocytes, heart smooth muscle cells, and trophoblasts. However, only on TEC CD105 is expressed in significant amounts that are relevant for immune recognition. CD105 is not confined to one tumor type, but has been reported to be expressed on TEC for a broad spectrum of solid tumors, e.g. breast, prostate, gastric, colorectal, renal and cervical tumors, and endometrial carcinoma, as well as for melanoma, and glioblastoma. However, binding affinity of CD105 is limited. In contrast to CD105, peptide epitopes selectively isolated from CD105 show high affinity to HLA-A and are beneficial targets for therapeutic interventions.

The term "human leukocyte antigen A2" (HLA-A2) as used herein refers to an HLA-A serotype that identifies the gene products of many HLA-A2 alleles, including HLA-A*0201, *0202, *0203, *0206, and *0207 gene products. HLA-A belongs to the superfamily of immunglobulins with a domain like structure, i.e. it is an MHC class I receptor consisting of an HLA-A heavy chain and β2-microglobulin. The heavy chain anchors the HLA complex in the membrane. HLA class I antigens are expressed on nearly all cells. HLA class I presents small allo-, auto- and inter-species antigens generated by cellular processing to the immune system, which surveys for non-self peptides. By interaction of HLA-A with T-cell receptors T-cell immune reactions are induced and regulated. As in most mammalian populations, MHC class I molecules are extremely variable in their primary structure, and HLA is ranked among the genes in humans with the fastest evolving coding sequence. Many diseases, like multiple sclerosis, schizophrenia or myasthenia gravis are associated with different HLA alleles.

Binding affinity of peptides according to the invention was controlled by a competitive binding assay. This assay is based on binding of the peptide to be tested and a reference peptide, e.g. a fluorescein-labeled reference peptide, to empty, acid-stripped HLA-A2-antigens expressed on cells. Reduction of binding of the reference peptide by competitive binding of different concentrations of the tested peptide to the cells, e.g. HLA-A2 positive cells JY, was analyzed by flow cytometry. The $IC_{50}$ value specifies the peptide concentration sufficient for 50% inhibition of binding of the reference

peptide, wherein $IC_{50} < 5$ $\mu$M means high affinity, $5$ $\mu$M $\leq IC_{50} \leq 15\mu$M means intermediate affinity, $15$ $\mu$M $< IC_{50} \leq 100$ $\mu$M means low affinity, and $IC_{50} > 100$ $\mu$M means no binding.

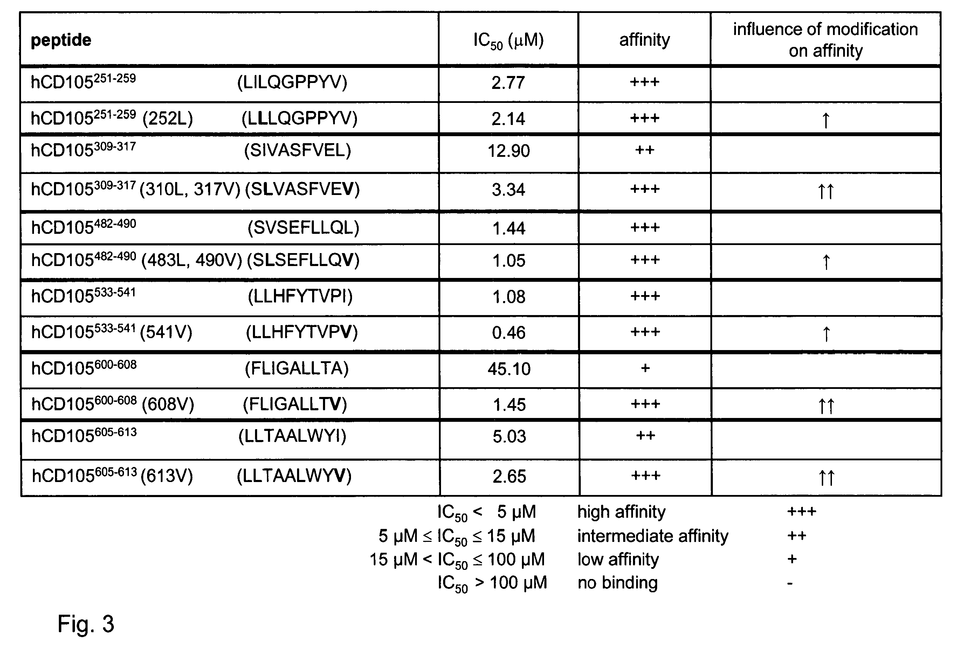

The present inventors surprisingly discovered that presentation of antigens by antigen presenting cells (APC) is enhanced for antigens, which possess a specific sequence motif of two or more essential amino acids, herein also called peptide anchor residues, in the context of a nonapeptide. Examples of essential amino acids of the specific sequence motif are leucine, methionine and valine. Amino acid exchanges in peptides improved binding to HLA-A2, when an amino acid at anchor position 2 was changed by leucine or methionine and/or an amino acid at anchor position 9 was changed by leucine or valine. The competitive binding assay indicates a binding affinity to HLA-A2 for CD105 derived peptides with leucine or methionine at amino acid position 2 and/or valine or leucine at amino acid position 9 by an $IC_{50}$ value below 5 $\mu$M (Fig. 3). An important advantage is that the recognition of the peptides by T cells was not altered by these amino acid exchanges. In other words, the recognition of the amended/modified/altered nonamer peptides of the invention by T cells is the same as with wild type/parental/unmodified peptides Leucine or methionine at anchor position 2 and/or valine or leucine at anchor position 9 of a specific binding motif in the context of a nonapeptide not only show high binding affinity of the peptides to HLA-A2, but also high immunogenicity and induction of T cell responses demonstrated by IFN-$\gamma$ ELISPOT, which measures the ability of T cells to respond to a peptide. Specific responses to CD105 derived peptides in peripheral blood of melanoma patients were significantly increased by the exchange of amino acids at amino acid position 2 against leucine and/or amino acid position 9 against valine in comparison to unchanged peptides (Fig. 5).

To analyze the immunogenicity of the peptides according to the invention *in vivo* HLA-A2/k$^b$ transgenic mice were vaccinated with the peptides derived from CD105 having a specific binding motif at amino acid position 2 and/or 9. HLA-A2/k$^b$ transgenic mice express a chimeric MHC class I antigen comprising peptide binding domains $\alpha$1 and $\alpha$2 of the human HLA-A2 antigen together with transmembrane, intracellular and $\alpha$3 domains of the murine H-2k$^b$ antigen allowing optimal interaction with murine T cells. In accordance to high affinity and high immune responses in melanoma patients' blood samples, high immune responses were induced by peptides with leucine at amino acid position 2 and/or valine at amino acid position 9 (Fig. 6). In comparison to parental CD105 derived peptides, immune responses were enhanced by exchanges of amino acids against leucine or methionine at anchor position 2 and/or against valine or leucine at anchor position 9.

Therefore, peptides with exchanged amino acids according to the invention optimize binding to HLA-A2 and enhance immunogenicity.

In contrast to tumor cells and adjacent neoplastic cells, tumor stromal cells are genetically stable. Therefore, peptides according to the invention are more suitable antigens for immunotherapy than antigens derived from tumor or neoplastic cells.

In a preferred embodiment of the invention the $IC_{50}$ is equal or below 3.5 $\mu$M.

In another preferred embodiment of the invention the peptide is selected from the group consisting of SEQ ID NO: 11 to SEQ ID NO: 16.

Binding affinities of peptides including SEQ ID NO: 11 to SEQ ID NO: 16 are indicated by an $IC_{50}$ equal or below 3.5 $\mu$M (Fig. 3), and specific responses to CD105 derived peptides in peripheral blood of melanoma patients were multiplied by using peptides of SEQ ID NO: 12, 15, and 16 in comparison to control experiments and in comparison to parental peptides selected from CD105 (Fig. 5). In accordance with improved affinity and immune response, enhanced immune responses were induced in melanoma patients' blood samples by peptides of SEQ ID NO: 12, 15, and 16 in comparison to control experiments and in comparison to parental epitopes in the mouse model (Fig. 6).

In another preferred embodiment of the invention the peptide is derived from mammalian endoglin, mice endoglin, rat endoglin, guinea pig endoglin and/or primate endoglin, particularly human endoglin.

As the human peptides SEQ ID NO: 15 and 16 are also included in the murine CD105 amino acid sequence (Fig. 7), major side effects of immune responses induced by polypeptides comprising peptides SEQ ID NO: 15 and/or 16 are excluded in HLA-A2/k$^b$ transgenic mouse model.

In a preferred embodiment of the invention the reference peptide is a fluorescein labeled peptide. In one possible embodiment the fluorescein-labeled reference peptide is (FLPSDC(FI)FPSV (JPT Peptide Technologies GmbH, Berlin, Germany). In a further preferred embodiment of the invention competitive binding assay is performed with HLA-A2 positive cells, preferably JY cells.

It is also an object of the invention to provide an artificial peptide for the treatment of cancer, the peptide is derived from human endoglin and binds specifically to human leukocyte antigen A2, wherein the artificial peptide is selected from the group consisting of SEQ ID NO: 1 to SEQ ID NO: 16.

In order to use CD105 as a target for tumor therapy distinct peptides have to be isolated that are immunogenic, function as epitopes and are presented on APC.

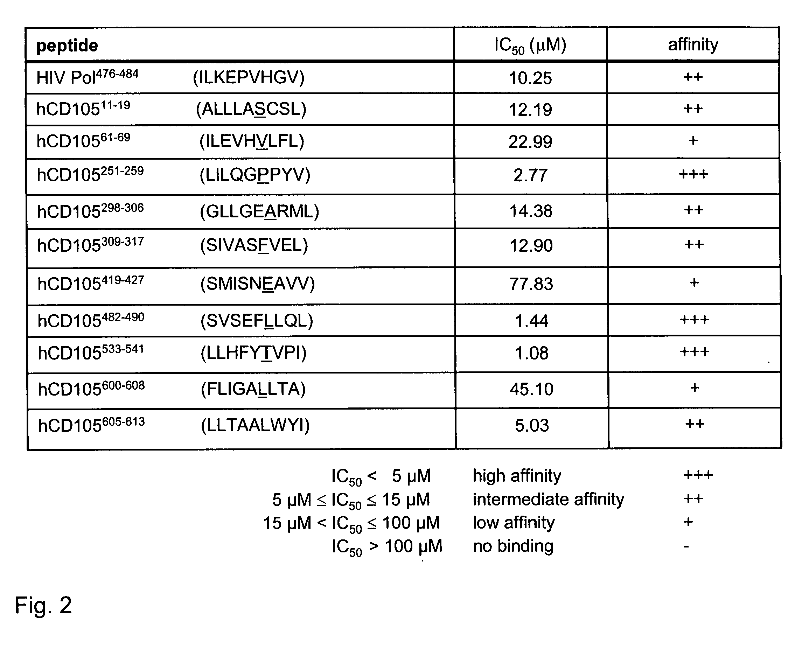

CD105 is a large polypeptide of 658 (L-endoglin) or 625 (S-endoglin) amino acids, from which specific peptides have to be chosen that possess the desired immunogenicity and capability to be presented on APC. By reverse immunology the present inventors have specifically selected peptides of SEQ ID NO: 1 to 10 from human CD105 that bind to the common MHC class I antigen HLA-A2. The selected peptides all bind to HLA, and binding affinity ranges between high affinity with an $IC_{50}$ value below 5 $\mu$M, intermediate affinity with an $IC_{50}$ equal or between 5 $\mu$M and 15 $\mu$M; and low affinity with an $IC_{50}$ between 15 $\mu$M and 100 $\mu$M or equal to 100 $\mu$M (Fig. 2). The peptides selected from human CD105 were further improved in affinity to HLA-A2. Peptides of SEQ ID NO: 11 to 16 indicate an $IC_{50}$ value below or equal to 3.5 $\mu$M, wherein amino acids at anchor position 2 of the nonapeptides of SEQ ID NO: 1 to 10 selected from CD105 were exchanged against methionine and leucine and/or amino acids at anchor position 9 were exchanged against valine or leucine (Fig. 3). Especially, peptides of SEQ ID NO: 3, 7, 8 and 11 to 16 show high binding affinity to HLA-A2.

Artificial peptides of SEQ ID NO: 1 to SEQ ID NO: 16 according to the invention not only bind specifically to HLA-A2, but also show high immunogenicity that was measured by detecting the ability of T cells to respond to a peptide according to the invention. Therefore, generation of functionally CD105 reactive T cells induced by peptides of SEQ ID NO: 1 to 16 was measured by IFN-$\gamma$ ELISPOT assays. Different concentrations of peripheral blood lymphocytes of a melanoma patient were prestimulated *in vitro* with peptides of SEQ ID NO: 1 to 16 and their reactivities to the respective peptides were analyzed by IFN-$\gamma$ ELISPOT. Specific immune responses to CD105 peptides in peripheral blood of melanoma patients were readily detectable for all peptides of SEQ ID NO: 1 to 16 (Figs. 4 and 5). Especially peptides of SEQ ID NO: 1, 3, 7, 8, 10, 12, 15 and 16 induce T cell activity *in vitro* very efficiently.

To analyze immunogenicity of peptides of SEQ ID NO: 1 to 16 *in vivo* HLAA2/k$^b$ transgenic mice were vaccinated with these peptides, and immune responses to peptides of SEQ ID NO: 1 to 16 are analyzed. As shown in Fig. 6 immunization of mice induced CD105 specific immune responses. Especially, peptides of SEQ ID NO: 3, 7, 8, 10, 12, 15 and 16 evoked high T cell IFN-$\gamma$ responses.

Another aspect of the invention concerns a polypeptide for the treatment of cancer, the polypeptide comprises an artificial peptide according to the invention.

Furthermore, the invention is directed to a nucleic acid for the treatment of cancer, the nucleic acid encodes at least one peptide or polypeptide according to the invention.

The term "nucleic acid" as used herein refers to a macromolecule composed of monomeric nucleotides, wherein nucleic acids include amongst other deoxynucleic acids (DNA); ribonucleic acids (RNA); artificial nucleic acids, like peptide nucleic acid (PNA) and glycolic nucleic acid (GNA); single stranded nucleic acids and double stranded nucleic acids. The term "nucleic acid" also comprises recombined nucleic acid as well as vectors carrying nucleic acids and/or recombined nucleic acids. The term "vector" refers to a vehicle for transferring genetic material into a cell, wherein plasmids, viral vectors, cloning vectors, expression vectors, transcription vectors, artificial particles and artificial chromosomes are included. The vector comprises double or single stranded nucleic acids as DNA or RNA and includes at least a transgene, a backbone and optionally a promoter and a marker.

In a preferred embodiment of the invention the nucleic acid comprises an expression system. By means of the expression system the peptides and/or polypeptides encoded by the nucleic acid can be expressed in an organism to be treated. In addition, the nucleic acid can be expressed in a system to gain therapeutic peptides, polypeptide or antibodies.

The invention also concerns an antibody against a peptide or polypeptide according to the invention for the treatment of cancer.

The term "antibody" as used herein refers to an immunoglobulin used by the immune system to identify and neutralize foreign objects. The term "antibody" includes monoclonal and polyclonal antibodies; antibodies of all known isotypes; artificial antibodies; and a functional part of an antibody.

Moreover, the invention relates to a vaccine comprising at least one substance selected from the group consisting of a peptide, a nucleic acid and an antibody according to the invention.

The data of affinity to HLA-A2 and immunogenicity of artificial peptides according to the invention suggest that passive immunotherapy using antibodies specific for artificial peptides according to the invention either alone or conjugated with different effector molecules as well as active immunotherapy by peptides according to the invention inhibit angiogenesis, tumor growth, and metastases. Antibodies specific for parental CD105 react only weakly or not at all with quiescent endothelium and side effects were low (Hofmeister et al., 2008). This property can also be applied to the peptides according to the invention.

In a preferred embodiment of the invention the vaccine is used for an active immunization. In another preferred embodiment the vaccine is used for a passive immunization. In a third embodiment the vaccine is used for DNA vaccination.

In a further preferred embodiment of the invention the vaccine comprises an adjuvant. The adjuvant includes, but is not limited to pharmaceutically acceptable carriers; co-stimulatory substances; dendritic cells; *Bacillus Calmette-Guéri;* molecules expressed by Epstein-Barr virus immortalized B cells, like B7.1 (DC80) and B7.2 (CD 86); GM-CSF; incomplete Freund's adjuvant; and cisplatin. Active immunization combined with co-stimulatory substances, like cisplatin revealed a synergistic antitumor activity.

In addition the invention is directed to a method for preparing a vaccine comprising the steps of:

a) providing the peptide, the polypeptide or the antibody according to the invention;
b) admixing an adjuvant to the peptide, polypeptide or antibody.

Furthermore, the invention concerns the use of at least one peptide, polypeptide, nucleic acid or antibody according to the invention for the manufacture of a drug and/or a vaccine for the treatment and/or prevention of cancer or an immunological disorder.

Manufactured vaccines and/or drugs according to the invention are employed to treat diseases, like cancer including non-Hodgkin's lymphoma; colorectal, head, neck, cervix, renal, breast, prostate, gastric and colorectal tumors; endometrial carcinoma; melanoma; and glioblastoma cancer. Immune deficiencies, such as X-linked agammaglobulinemia and hypogammaglobulinemia resulting in partial or complete lack of antibodies can also be treated by a vaccine and/or a drug according to the invention.

**Material and methods**

**1. Cells and cell lines**

Human HLA-A*0201-positive cell lines T2, a transporter associated with antigen processing (TAP)-deficient T cell leukemia/B cell line hybrid and JY, a B-LCL were cultured in RPMI 1640 supplemented with 10% heat inactivated fetal calve serum (FCS). After informed consent peripheral blood lymphocytes (PBL) were collected from HLA-A2-positive patients with advanced malignant melanoma. PBL from HLA-A2-positive healthy individuals were used as controls. PBL were isolated using Lymphoprep separation (Axis-Shield PoC AS, Oslo, Norway) according to the manufacturer's instructions and used directly or frozen in FCS with 10% DMSO. PBL were cultured in RPMI/10% human AB serum.

**2. Immunohistochemical staining**

Paraffin sections were treated with pronase (Roche, Mannheim, Germany) for 20 minutes at 37°C and stained with the CD105-specific antibody SN6h (Acris Antibodies GmbH, Hiddenhausen, Germany) and the Vector VIP system (Vector Laboratories, Burlingame, USA) according to the manufacturer's instructions.

**3. Selection of CD105 nonamer peptides**

Peptides derived from the full-length human CD105 polypeptide were selected using both BIMAS (Parker et al., 1994) and SYFPEITHI (Rammensee et al., 1999) peptide binding algorithms available via the internet (http://bimas.cit.nih.gov/molbio/hla_bind/) and (http://www.syfpeithi.de/). Residues 2 and 9 of some peptides were changed to optimize anchor amino acids.

**4. Competitive binding assay for binding to HLA-A2 molecules**

The binding affinity of the synthetic peptides (GeneScript Corporation, Piscataway, New Jersey, USA) to HLA-A2 molecules was measured in the competitive binding assay as described previously (Kessler et al., 2003). The assay is based on the binding of the competitive peptide to be tested and the fluorescein-labeled reference peptide (FLPSDC (Fl)FPSV, JPT Peptide Technologies GmbH, Berlin, Germany) to the acid-stripped HLA-A2 positive cell line JY. Reduction of the binding of the reference peptide was analyzed by flow cytometry. The percentage of binding inhibition of the Fl-labeled reference peptide was calculated using the following formula:

$$(1-(MF_{reference + competitor\ peptide} - MF_{background})/(MF_{reference\ peptide} - MF_{background})) \times 100\%$$

The binding affinities of the competitor peptides are expressed as $IC_{50}$ values in the figures, specifying their concentration sufficient for 50% inhibition of binding of the reference peptide. $IC_{50}$ was calculated by nonlinear regression analysis with software CurveExpert 1.3. Peptides with an $IC_{50} < 5\ \mu M$ were considered as high-affinity, with $5\ \mu M \leq IC_{50} \leq 15\ \mu M$ as intermediate-affinity, with $15\ \mu M < IC_{50} \leq 100\ \mu M$ as low-affinity, and with $IC_{50} > 100\ \mu M$ as no binders.

**5. Antigen stimulation of PBL**

To extend the sensitivity of the ELISPOT assay, PBL were stimulated once *in vitro* before analysis (McCutcheon et al., 1997). At day 0, PBL were thawed or freshly isolated from peripheral blood and plated in a cell concentration of $1 \times 10^6$/ml and 5 ml/well in 6-well plates (Greiner GmbH, Frickenhausen, Germany) in X-vivo medium (Cambrex Biosciences, Verviers, Belgium) with 10% heat inactivated human AB serum in the presence of 10 μM peptide (GeneScript Corporation, Piscataway, New Jersey, USA). On day 1 and 4 40 U IL-2/ml (Proleukin, Chiron GmbH, Munich, Gemany) were added to the culture. The cultured cells were tested for reactivity in ELISPOT on day 7.

### 6. ELISPOT assays

The human IFN-γ ELISPOT assay was used to quantify peptide epitope-specific IFN-γ-releasing effector cells as described in Berke *et al.* 2000. Nitrocellulose-bottomed 96-well plates (MultiScreen MAIP N45, Millipore GmbH, Schwalbach, Germany) were activated with 35% ethanol, washed with PBS and coated with anti-IFN-γ antibody (1-D1 K, Mabtech, Hamburg, Germany). The wells were washed and blocked by X-vivo medium before adding $1 \times 10^4$ stimulator T2 cells loaded with or without 10 μM peptide and $3 \times 10^5$, $1 \times 10^5$ or $3 \times 10^4$ effector cells. After incubation overnight the wells were washed before addition of biotinylated secondary antibody (7-B6-1-Biotin, Mabtech, Hamburg, Germany). The plates were incubated for 2 h, washed, and streptavidine-enzyme conjugate (Streptavidin-ALP-PQ, Mabtech, Hamburg, Germany) was added. Incubation at room temperature for 1 h was followed by addition of enzyme substrate NBT/BCIP (Mabtech, Hamburg, Germany). The reaction was stopped by washing with tap water upon the appearance of dark purple spots. Spots were counted using the ImmunoSpot Series 2.0 Analyzer (CTL Cellular Technology Ltd., Schwäbisch Gmünd, Germany). The peptide-specific CTL frequency was calculated from the numbers of spot-forming cells. All assays were performed in duplicates. Responders were defined as having an average number of > 25 antigen-specific spots per $10^5$ Cells (Spots $_{\text{with peptide}}$ - Spots $_{\text{without peptide}}$).

### 7. Immunization of mice and analysis of immune responses by ELISPOT

HLA-A2/k$^b$ transgenic mice (deposited by Linda A. Sherman at MMRCC, University of North Carolina, Chapel Hill, USA) were immunized with a mixture of 5-6 CD105 peptides (5 μg/peptide) and incomplete Freund's adjuvant 7 times once weekly. Spleen cells were analyzed in a murine IFN-γ ELISPOT after *in vitro* stimulation. Cells were stimulated for six days with 200 U IL-2/ml and 10 μM peptide loaded, mitomycin C treated LPS blasts, generated by stimulation of HLA-A2k$^b$ spleen cells with 25 μg/ml LPS (Sigma-Aldrich, Taufkirchen, Germany) and 7 μg/ml dextran sulfate for three days. Subsequently $3 \times 10^5$, $1 \times 10^5$, and $0.3 \times 10^5$ spleen cells were seeded with or without 10 μM peptide in RPMI 1640/10% FCS in duplicates in nitrocellulose-bottomed 96-well plates (MultiScreen MAIP N45, Millipore GmbH) coated with an IFN-γ specific coating antibody (Mabtech) and blocked with cell culture medium and incubated overnight. The development of the murine IFN-γ ELISPOT was done as described for the human ELISPOT, with the exception that PBS/0.05% Tween 20 was used as washing buffer and the biotinylated anti-mouse IFN-γ antibody (Mabtech, diluted in PBS/1% BSA) as detection antibody.

**Table 1: Sequence listing**

| SEQ ID NO: | Sequence name | Additional remarks |
|---|---|---|
| 1. | hCD105 11-19 | parental peptide; AA position 11-19 |
| 2. | hCD105 61-69 | parental peptide; AA position 61-69 |
| 3. | hCD105 251-259 | parental peptide; AA position 251-259 |
| 4. | hCD105 298-306 | parental peptide; AA position 298-306 |
| 5. | hCD105 309-317 | parental peptide; AA position 309-317 |
| 6. | hCD105 419-427 | parental peptide; AA position 419-427 |
| 7. | hCD105 482-490 | parental peptide; AA position 482-490 |
| 8. | hCD105 533-541 | parental peptide; AA position 533-541 |
| 9. | hCD105 600-608 | parental peptide; AA position 600-608 |
| 10. | hCD105 605-613 | parental peptide; AA position 605-613 |
| 11. | hCD105 251-259 (252L) | AA 252: leucine |
| 12. | hCD105 309-317 (310L, 317V) | AA 310: leucine AA 317: valine |
| 13. | hCD105 482-490 (483L, 490V) | AA 483: leucine AA 490: valine |

(continued)

| SEQ ID NO: | Sequence name | Additional remarks |
|---|---|---|
| 14. | hCD105 533-541 (541V) | AA541: valine |
| 15. | hCD105 600-608 (608V) | AA 608: valine |
| 16. | hCD105 605-613 (613V) | AA 613: valine |
| 17. | hCD105 L-isoform | parental |
| 18. | hCD105 S-isoform | parental |
| 19. | murine CD1 05 | parental |
| AA = amino acid; L= leucine; V = valine | | |

**References**

Adami et al. (2003) Cancer risk following organ transplantation: a nationwide cohort study in Sweden. Br J Cancer 89: 1221-1227.

Berke et al. (2000) Peptides spanning the junctional region of both the abl/bcr and the bcr/abl fusion proteins bind common HLA class I molecules. Leukemia 14: 419-426.

Dales et al. (2004) Prognostic significance of angiogenesis evaluated by CD105 expression compared to CD31 in 905 breast carcinomas: correlation with long-term patient outcome. Int J Oncol 24: 1197-1204.

Hofmeister et al. (2008) Anti-cancer therapies targeting the tumor stroma. Cancer Immunol. Immunother. 57: 1-17.

Kessler et al. (2003) Competition-based cellular peptide binding assays for 13 prevalent HLA class I alleles using fluorescein-labeled synthetic peptides. Human Immunology 64: 245-255.

Kondratiev et al. (2004) Intratumoral CD8+ T Lymphocytes as a prognostic factor of survival in endometrial carcinoma. Clin Cancer Res 10: 4450-4456.

McCutcheon et al. (1997) A sensitive ELISPOT assay to detect low-frequency human T lymphocytes. Journal of Immunological Methods 210: 149-166.

Parker et al. (1994) Scheme for ranking potential HLA-A2 binding peptides based on independent binding of individual peptide side-chains. J Immunol 152: 163-175.

Rammensee et al. (1999) SYFPEITHI: database for MHC ligands and peptide motifs. Immunogenetics 50: 213-219.

**Claims**

1. An artificial peptide for the treatment of cancer, the peptide is derived from endoglin and comprises a nonapeptide, which inhibits 50 % of binding of a reference peptide to human leukocyte antigen A2 in a competitive binding assay at a concentration below 5 µM of the nonapeptide, wherein the nonapeptide has a leucine or methionine at amino acid position 2 and/or a valine or leucine at amino acid position 9.

2. The artificial peptide of claim 1, wherein the peptide is selected from the group consisting of SEQ ID NO: 11 to SEQ ID NO: 16.

3. The artificial peptide of claim 1 or 2, wherein the peptide is derived from mammalian endoglin, mice endoglin, rat endoglin, guinea pig endoglin and/or primate endoglin, particularly human endoglin.

4. The artificial peptide of any of the preceding claims, wherein the reference peptide is a fluorescein labeled peptide.

5. An artificial peptide for the treatment of cancer, the peptide is derived from human endoglin and binds specifically to human leukocyte antigen A2, wherein the peptide is selected from the group consisting of SEQ ID NO: 1 to SEQ ID NO: 16.

6. A polypeptide for the treatment of cancer, the polypeptide comprises the artificial peptide of any of the preceding claims.

7. A nucleic acid for the treatment of cancer, the nucleic acid encodes at least one peptide of any of claims 1 to 5 or at least one polypeptide of claim 6.

8. An antibody against at least one peptide of any of claims 1 to 5 or at least one polypeptide of claim 6 for the treatment of cancer.

9. A vaccine comprising at least one substance selected from the group consisting of the peptide of any of claims 1 to 5, the polypeptide of claim 6, the nucleic acid of claim 7 and the antibody of claim 8.

10. A method for preparing a vaccine comprising the steps of:

    a) providing the peptide of any of claims 1 to 5, the polypeptide of claim 6 or the antibody of claim 8;
    b) admixing an adjuvant to the peptide, polypeptide or antibody.

11. Use of at least one peptide of any of claims 1 to 5, polypeptide of claim 6, nucleic acid of claim 7 or antibody of claim 8 for the manufacture of a drug and/or a vaccine for the treatment and/or prevention of cancer or an immunological disorder.

EP 2 153 845 A1

⌐signal sequence (25 aa)          ⌐extracellular domain (561 aa)

MDRGTLPLAV<u>ALLLASCSL</u>SPTSLAETVHCDLQPVGPERDEVTYTTSQVSKGCVAQA

PNAI<u>LEVHVLFL</u>EFPTGPSQLELTLQASKQNGTWPREVLLVLSVNSSVFLHLQALGIPL

HLAYNSSLVTFQEPPGVNTTELPSFPKTQILEWAAERGPITSAAELNDPQSILLRLGQA

QGSLSFCMLEASQDMGRTLEWRPRTPALVRGCHLEGVAGHKEAHILRVLPGHSAGP

RTVTVKVELSCAPGDLDAV<u>LILQGPPYV</u>SWLIDANHNMQIWTTGEYSFKIFPEKNIRGF

KLPDTPQ<u>GLLGEARML</u>NA<u>SIVASFVEL</u>PLASIVSLHASSCGGRLQTSPAPIQTTPPKDT

CSPELLMSLIQTKCADDAMTLVLKKELVAHLKCTITGLTFWDPSCEAEDRGDKFVLRS

AYSSCGMQVSA<u>SMISNEA</u>VVNILSSSSPQRKKVHCLNMDSLSFQLGLYLSPHFLQAS

NTIEPGQQSFVQVRVSP<u>SVSEFLLQL</u>DSCHLDLGPEGGTVELIQGRAAKGNCVSLLS

PSPEGDPRFSF<u>LLHFYTVPI</u>PKTGTLSCTVALRPKTGSQDQEVHRTVFMRLN


          ⌐TM domain (25 aa)          ⌐cytoplas. domain
                                          (L-isoform 47 aa, S-isoform 14 aa)

IISPDLSGCTSKGLVLPAVLGITFGA<u>FLIGALLTA</u>ALWYIYSHTR

-SPSKREPVVAVAAPASSESSSTNHSIGSTQSTPCSTSSMA          L-isoform

-EYPRPPQ                                            S-isoform


Fig. 1

| peptide | | IC$_{50}$ (µM) | affinity |
|---|---|---|---|
| HIV Pol[476-484] | (ILKEPVHGV) | 10.25 | ++ |
| hCD105[11-19] | (ALLLASCSL) | 12.19 | ++ |
| hCD105[61-69] | (ILEVHVLFL) | 22.99 | + |
| hCD105[251-259] | (LILQGPPYV) | 2.77 | +++ |
| hCD105[298-306] | (GLLGEARML) | 14.38 | ++ |
| hCD105[309-317] | (SIVASFVEL) | 12.90 | ++ |
| hCD105[419-427] | (SMISNEAVV) | 77.83 | + |
| hCD105[482-490] | (SVSEFLLQL) | 1.44 | +++ |
| hCD105[533-541] | (LLHFYTVPI) | 1.08 | +++ |
| hCD105[600-608] | (FLIGALLTA) | 45.10 | + |
| hCD105[605-613] | (LLTAALWYI) | 5.03 | ++ |

$$IC_{50} < \ 5 \ \mu M \qquad \text{high affinity} \qquad +++$$
$$5 \ \mu M \leq IC_{50} \leq 15 \ \mu M \qquad \text{intermediate affinity} \qquad ++$$
$$15 \ \mu M < IC_{50} \leq 100 \ \mu M \qquad \text{low affinity} \qquad +$$
$$IC_{50} > 100 \ \mu M \qquad \text{no binding} \qquad -$$

Fig. 2

| peptide | | IC$_{50}$ (µM) | affinity | influence of modification on affinity |
|---|---|---|---|---|
| hCD105[251-259] | (LILQGPPYV) | 2.77 | +++ | |
| hCD105[251-259] (252L) | (LLLQGPPYV) | 2.14 | +++ | ↑ |
| hCD105[309-317] | (SIVASFVEL) | 12.90 | ++ | |
| hCD105[309-317] (310L, 317V) | (SLVASFVEV) | 3.34 | +++ | ↑↑ |
| hCD105[482-490] | (SVSEFLLQL) | 1.44 | +++ | |
| hCD105[482-490] (483L, 490V) | (SLSEFLLQV) | 1.05 | +++ | ↑ |
| hCD105[533-541] | (LLHFYTVPI) | 1.08 | +++ | |
| hCD105[533-541] (541V) | (LLHFYTVPV) | 0.46 | +++ | ↑ |
| hCD105[600-608] | (FLIGALLTA) | 45.10 | + | |
| hCD105[600-608] (608V) | (FLIGALLTV) | 1.45 | +++ | ↑↑ |
| hCD105[605-613] | (LLTAALWYI) | 5.03 | ++ | |
| hCD105[605-613] (613V) | (LLTAALWYV) | 2.65 | +++ | ↑↑ |

| | | |
|---|---|---|
| IC$_{50}$ < 5 µM | high affinity | +++ |
| 5 µM ≤ IC$_{50}$ ≤ 15 µM | intermediate affinity | ++ |
| 15 µM < IC$_{50}$ ≤ 100 µM | low affinity | + |
| IC$_{50}$ > 100 µM | no binding | - |

Fig. 3

Fig. 4

Fig. 5

Fig. 6

EP 2 153 845 A1

⌐signal sequence (26 aa)         ⌐extracellular domain (555 aa)

MDRGVLPLPITLLFVIYSFVPTTGLAERVGCDLQPVDPTRGEVTFTTSQVSEGCVAQAANA
VREVHVLFLDFPGMLSHLELTLQASKQNGTETREVFLVLVSNKNVFVKFQAPEIPLHLAYD
SSLVIFQGQPRVNITVLPSLTSRKQILDWAATKGAITSIAALDDPQSIVLQLGQDPKAPFLCL
PEAHKDMGATLEWQPRAQTPVQSCRLEGVSGHKEAYILRILPGSEAGPRTVTVMMELSC
TSGDAILILHGPPYVSWFIDINHSMQILTTGEYSVKIFPGSKVKGVELPDTPQGLIAEARKLN
ASIVTSFVELPLVSNVSLRASSCGGVFQTTPAPVVTTPPKDTCSPVLLMSLIQPKCGNQVMT
LALNKKHVQTLQCTITGLTFWDSSCQAEDTDDHLVLSSAYSSCGMKVTAHVVSNEVIISFPS
GSPPLRKKVQCIDMDSLSFQLGLYLSPHFLQASNTIELGQQAFVQVSVSPLTSEVTVQLDSC
HLDLGPEGDMVELIQSRTAKGSCVTLLSPSPEGDPRFSFLLRVYMVPTPTAGTLSCNLALRP

                   ⌐TM domain (25 aa)           ⌐ cytoplas. domain

STLSQEVYKTVSMRLNVVSPDLSGKGLVLPSVLGITFGAFLIGALLTAALWYIYSHTRGPSKR
EPVVAVAAPASSESSSTNHSIGSTQSTPCSTSSMA       ————

Fig. 7

| human parental CD105 peptide | human affinity improved CD105 peptide |
|---|---|
| hCD105[11-19]　　(ALLLASCSL) | |
| hCD105[61-69]　　(ILEVHVLFL) | |
| hCD105[251-259]　(LILQGPPYV) | hCD105[251-259] (252L)　　　(LLLQGPPYV) |
| hCD105[298-306]　(GLLGEARML) | |
| hCD105[309-317]　(SIVASFVEL) | hCD105[309-317] (310L,317V)　(SLVASFVEV) |
| hCD105[419-427]　(SMISNEAVV) | |
| hCD105[482-490]　(SVSEFLLQL) | hCD105[482-490] (483L,490V)　(SLSEFLLQV) |
| hCD105[533-541]　(LLHFYTVPI) | hCD105[533-541] (541V)　　　(LLHFYTVPV) |
| hCD105[600-608]　(FLIGALLTA) | hCD105[600-608] (608V)　　　(FLIGALLTV) |
| hCD105[605-613]　(LLTAALWYI) | hCD105[605-613] (613V)　　　(LLTAALWYV) |

Fig. 8

Europäisches
Patentamt

European
Patent Office

Office européen
des brevets

# EUROPEAN SEARCH REPORT

**Application Number**

EP 08 01 4455

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TAN GUANG-HONG ET AL: "Active immunotherapy of tumors with a recombinant xenogeneic endoglin as a model antigen" EUROPEAN JOURNAL OF IMMUNOLOGY, vol. 34, no. 7, July 2004 (2004-07), pages 2012-2021, XP002508237 ISSN: 0014-2980 * abstract * * page 2013, column 1, last paragraph - column 2, paragraph 4 * * page 2015, column 1, paragraph 2 - last paragraph * * page 2018, column 1, paragraph 2 * * page 2018, column 2 - page 2019, column 1, paragraph 2 * ----- | 1-3,6, 9-11 | INV. A61K39/00 C07K16/28 A61P35/00 |
| X | TAN GUANG-HONG ET AL: "Combination of recombinant xenogeneic endoglin DNA and protein vaccination enhances anti-tumor effects" IMMUNOLOGICAL INVESTIGATIONS, vol. 36, no. 4, 2007, pages 423-440, XP008099601 ISSN: 0882-0139 * abstract * * page 425, paragraph 2 - page 426, paragraph 1 * * page 428, paragraphs 2,3 * ----- | 1-3,6,7, 9-11 | |
| X | MIZUTANI N ET AL: "DNA vaccines suppress angiogenesis and protect against growth of breast cancer metastases." BREAST DISEASE 2004, vol. 20, 2004, pages 81-91, XP008099602 ISSN: 0888-6008 * page 87, column 1, last paragraph - page 89, column 1, paragraph 1 * ----- -/-- | 7,9,11 | TECHNICAL FIELDS SEARCHED (IPC) A61K |

The present search report has been drawn up for all claims

| Place of search | Date of completion of the search | Examiner |
|---|---|---|
| The Hague | 16 December 2008 | Noë, Veerle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European
Patentamt

European
Patent Office

Office européen
des brevets

**EUROPEAN SEARCH REPORT**

Application Number

EP 08 01 4455

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JIAO ET AL: "A plasmid DNA vaccine encoding the extracellular domain of porcine endoglin induces anti-tumour immune response against self-endoglin-related angiogenesis in two liver cancer models" DIGESTIVE AND LIVER DISEASE, W.B. SAUNDERS, vol. 38, no. 8, 1 August 2006 (2006-08-01), pages 578-587, XP005541415 ISSN: 1590-8658 * abstract * | 7,9,11 | |
| X | US 6 190 660 B1 (SEON BEN K [US]) 20 February 2001 (2001-02-20) * abstract * * column 4, lines 53-64 * * column 5, lines 5-18 * * column 9, lines 26-33 * * page 9, line 52 - page 10, line 30 * * column 11, lines 19-51 * * column 19, line 63 - column 21, last line * | 8-11 | |
| A | HOFMEISTER V ET AL: "Peptide Epitopes derived from tumor stroma-associated antigens CD105 and FAP alpha for cancer immunotherapy" JOURNAL OF INVESTIGATIVE DERMATOLOGY, vol. 125, no. 3, Suppl. S, September 2005 (2005-09), page A70, XP008099590 & 35TH ANNUAL MEETING OF THE EUROPEAN-SOCIETY-FOR-DERMATOLOGICAL-RESEARCH; TUBINGEN, GERMANY; SEPTEMBER 22 -24, 2005 ISSN: 0022-202X * abstract * | | TECHNICAL FIELDS SEARCHED (IPC) |

The present search report has been drawn up for all claims

| Place of search | Date of completion of the search | Examiner |
|---|---|---|
| The Hague | 16 December 2008 | Noë, Veerle |

EPO FORM 1503 03.82 (P04C01)

EP 2 153 845 A1

**ANNEX TO THE EUROPEAN SEARCH REPORT
ON EUROPEAN PATENT APPLICATION NO.**

EP 08 01 4455

This annex lists the patent family members relating to the patent documents cited in the above-mentioned European search report.
The members are as contained in the European Patent Office EDP file on
The European Patent Office is in no way liable for these particulars which are merely given for the purpose of information.

16-12-2008

| Patent document cited in search report | Publication date | Patent family member(s) | Publication date |
|---|---|---|---|
| US 6190660 B1 | 20-02-2001 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Adami et al.** Cancer risk following organ transplantation: a nationwide cohort study in Sweden. *Br J Cancer,* 2003, vol. 89, 1221-1227
- **Berke et al.** Peptides spanning the junctional region of both the abl/bcr and the bcr/abl fusion proteins bind common HLA class I molecules. *Leukemia,* 2000, vol. 14, 419-426
- **Dales et al.** Prognostic significance of angiogenesis evaluated by CD105 expression compared to CD31 in 905 breast carcinomas: correlation with long-term patient outcome. *Int J Oncol,* 2004, vol. 24, 1197-1204
- **Hofmeister et al.** Anti-cancer therapies targeting the tumor stroma. *Cancer Immunol. Immunother.,* 2008, vol. 57, 1-17
- **Kessler et al.** Competition-based cellular peptide binding assays for 13 prevalent HLA class I alleles using fluorescein-labeled synthetic peptides. *Human Immunology,* 2003, vol. 64, 245-255
- **Kondratiev et al.** Intratumoral CD8+ T Lymphocytes as a prognostic factor of survival in endometrial carcinoma. *Clin Cancer Res,* 2004, vol. 10, 4450-4456
- **McCutcheon et al.** A sensitive ELISPOT assay to detect low-frequency human T lymphocytes. *Journal of Immunological Methods,* 1997, vol. 210, 149-166
- **Parker et al.** Scheme for ranking potential HLA-A2 binding peptides based on independent binding of individual peptide side-chains. *J Immunol,* 1994, vol. 152, 163-175
- **Rammensee et al.** SYFPEITHI: database for MHC ligands and peptide motifs. *Immunogenetics,* 1999, vol. 50, 213-219

SEQUENCE LISTING

<110> Julius-Maximilians-Universität Würzburg

<120> Endoglin peptides, vaccines and methods for preparing the same

<130> U30026

<160> 19

<170> PatentIn version 3.3

<210> 1
<211> 9
<212> PRT
<213> Homo sapiens

<400> 1

Ala Leu Leu Leu Ala Ser Cys Ser Leu
1               5


<210> 2
<211> 9
<212> PRT
<213> Homo sapiens

<400> 2

Ile Leu Glu Val His Val Leu Phe Leu
1               5


<210> 3
<211> 9
<212> PRT
<213> Homo sapiens

<400> 3

Leu Ile Leu Gln Gly Pro Pro Tyr Val
1               5


<210> 4
<211> 9
<212> PRT
<213> Homo sapiens

<400> 4

Gly Leu Leu Gly Glu Ala Arg Met Leu
1               5


<210> 5
<211> 9
<212> PRT
<213> Homo sapiens

<400> 5

Ser Ile Val Ala Ser Phe Val Glu Leu
1                   5


<210>  6
<211>  9
<212>  PRT
<213>  Homo sapiens

<400>  6

Ser Met Ile Ser Asn Glu Ala Val Val
1                   5


<210>  7
<211>  9
<212>  PRT
<213>  Homo sapiens

<400>  7

Ser Val Ser Glu Phe Leu Leu Gln Leu
1                   5


<210>  8
<211>  9
<212>  PRT
<213>  Homo sapiens

<400>  8

Leu Leu His Phe Tyr Thr Val Pro Ile
1                   5


<210>  9
<211>  9
<212>  PRT
<213>  Homo sapiens

<400>  9

Phe Leu Ile Gly Ala Leu Leu Thr Ala
1                   5


<210>  10
<211>  9
<212>  PRT
<213>  Homo sapiens

<400>  10

Leu Leu Thr Ala Ala Leu Trp Tyr Ile
1                   5


<210>  11
<211>  9
<212>  PRT
<213>  Homo sapiens

```
<400>  11

Leu Leu Leu Gln Gly Pro Pro Tyr Val
1                5


<210>  12
<211>  9
<212>  PRT
<213>  Homo sapiens

<400>  12

Ser Leu Val Ala Ser Phe Val Glu Val
1                5


<210>  13
<211>  9
<212>  PRT
<213>  Homo sapiens

<400>  13

Ser Leu Ser Glu Phe Leu Leu Gln Val
1                5


<210>  14
<211>  9
<212>  PRT
<213>  Homo sapiens

<400>  14

Leu Leu His Phe Tyr Thr Val Pro Val
1                5


<210>  15
<211>  9
<212>  PRT
<213>  Homo sapiens

<400>  15

Phe Leu Ile Gly Ala Leu Leu Thr Val
1                5


<210>  16
<211>  9
<212>  PRT
<213>  Homo sapiens

<400>  16

Leu Leu Thr Ala Ala Leu Trp Tyr Val
1                5


<210>  17
```

```
<211>    658
<212>    PRT
<213>    Homo sapiens

<400>    17

Met Asp Arg Gly Thr Leu Pro Leu Ala Val Ala Leu Leu Leu Ala Ser
1               5                   10                  15


Cys Ser Leu Ser Pro Thr Ser Leu Ala Glu Thr Val His Cys Asp Leu
                20                  25                  30


Gln Pro Val Gly Pro Glu Arg Asp Glu Val Thr Tyr Thr Thr Ser Gln
                35                  40                  45


Val Ser Lys Gly Cys Val Ala Gln Ala Pro Asn Ala Ile Leu Glu Val
        50                  55                  60


His Val Leu Phe Leu Glu Phe Pro Thr Gly Pro Ser Gln Leu Glu Leu
65                  70                  75                  80


Thr Leu Gln Ala Ser Lys Gln Asn Gly Thr Trp Pro Arg Glu Val Leu
                85                  90                  95


Leu Val Leu Ser Val Asn Ser Ser Val Phe Leu His Leu Gln Ala Leu
                100                 105                 110


Gly Ile Pro Leu His Leu Ala Tyr Asn Ser Ser Leu Val Thr Phe Gln
            115                 120                 125


Glu Pro Pro Gly Val Asn Thr Thr Glu Leu Pro Ser Phe Pro Lys Thr
            130                 135                 140


Gln Ile Leu Glu Trp Ala Ala Glu Arg Gly Pro Ile Thr Ser Ala Ala
145                 150                 155                 160


Glu Leu Asn Asp Pro Gln Ser Ile Leu Leu Arg Leu Gly Gln Ala Gln
                165                 170                 175


Gly Ser Leu Ser Phe Cys Met Leu Glu Ala Ser Gln Asp Met Gly Arg
                180                 185                 190


Thr Leu Glu Trp Arg Pro Arg Thr Pro Ala Leu Val Arg Gly Cys His
            195                 200                 205


Leu Glu Gly Val Ala Gly His Lys Glu Ala His Ile Leu Arg Val Leu
            210                 215                 220


Pro Gly His Ser Ala Gly Pro Arg Thr Val Thr Val Lys Val Glu Leu
```

225                     230                     235                     240

Ser Cys Ala Pro Gly Asp Leu Asp Ala Val Leu Ile Leu Gln Gly Pro
                245                 250                 255

Pro Tyr Val Ser Trp Leu Ile Asp Ala Asn His Asn Met Gln Ile Trp
            260                 265                 270

Thr Thr Gly Glu Tyr Ser Phe Lys Ile Phe Pro Glu Lys Asn Ile Arg
        275                 280                 285

Gly Phe Lys Leu Pro Asp Thr Pro Gln Gly Leu Leu Gly Glu Ala Arg
        290                 295                 300

Met Leu Asn Ala Ser Ile Val Ala Ser Phe Val Glu Leu Pro Leu Ala
305             310                 315                 320

Ser Ile Val Ser Leu His Ala Ser Ser Cys Gly Gly Arg Leu Gln Thr
            325                 330                 335

Ser Pro Ala Pro Ile Gln Thr Thr Pro Pro Lys Asp Thr Cys Ser Pro
            340                 345                 350

Glu Leu Leu Met Ser Leu Ile Gln Thr Lys Cys Ala Asp Asp Ala Met
            355                 360                 365

Thr Leu Val Leu Lys Lys Glu Leu Val Ala His Leu Lys Cys Thr Ile
        370                 375                 380

Thr Gly Leu Thr Phe Trp Asp Pro Ser Cys Glu Ala Glu Asp Arg Gly
385             390                 395                 400

Asp Lys Phe Val Leu Arg Ser Ala Tyr Ser Ser Cys Gly Met Gln Val
                405                 410                 415

Ser Ala Ser Met Ile Ser Asn Glu Ala Val Val Asn Ile Leu Ser Ser
            420                 425                 430

Ser Ser Pro Gln Arg Lys Lys Val His Cys Leu Asn Met Asp Ser Leu
            435                 440                 445

Ser Phe Gln Leu Gly Leu Tyr Leu Ser Pro His Phe Leu Gln Ala Ser
        450                 455                 460

Asn Thr Ile Glu Pro Gly Gln Gln Ser Phe Val Gln Val Arg Val Ser
465             470                 475                 480

14

```
Pro Ser Val Ser Glu Phe Leu Leu Gln Leu Asp Ser Cys His Leu Asp
            485                 490                 495

Leu Gly Pro Glu Gly Gly Thr Val Glu Leu Ile Gln Gly Arg Ala Ala
            500                 505                 510

Lys Gly Asn Cys Val Ser Leu Leu Ser Pro Ser Pro Glu Gly Asp Pro
        515                 520                 525

Arg Phe Ser Phe Leu Leu His Phe Tyr Thr Val Pro Ile Pro Lys Thr
        530                 535                 540

Gly Thr Leu Ser Cys Thr Val Ala Leu Arg Pro Lys Thr Gly Ser Gln
545                 550                 555                 560

Asp Gln Glu Val His Arg Thr Val Phe Met Arg Leu Asn Ile Ile Ser
            565                 570                 575

Pro Asp Leu Ser Gly Cys Thr Ser Lys Gly Leu Val Leu Pro Ala Val
            580                 585                 590

Leu Gly Ile Thr Phe Gly Ala Phe Leu Ile Gly Ala Leu Leu Thr Ala
            595                 600                 605

Ala Leu Trp Tyr Ile Tyr Ser His Thr Arg Ser Pro Ser Lys Arg Glu
        610                 615                 620

Pro Val Val Ala Val Ala Ala Pro Ala Ser Ser Glu Ser Ser Ser Thr
625                 630                 635                 640

Asn His Ser Ile Gly Ser Thr Gln Ser Thr Pro Cys Ser Thr Ser Ser
            645                 650                 655

Met Ala
```

```
<210>  18
<211>  625
<212>  PRT
<213>  Homo sapiens

<400>  18
```

```
Met Asp Arg Gly Thr Leu Pro Leu Ala Val Ala Leu Leu Leu Ala Ser
1               5                 10                  15

Cys Ser Leu Ser Pro Thr Ser Leu Ala Glu Thr Val His Cys Asp Leu
        20                  25                  30
```

```
Gln Pro Val Gly Pro Glu Arg Asp Glu Val Thr Tyr Thr Thr Ser Gln
        35              40              45

Val Ser Lys Gly Cys Val Ala Gln Ala Pro Asn Ala Ile Leu Glu Val
        50              55              60

His Val Leu Phe Leu Glu Phe Pro Thr Gly Pro Ser Gln Leu Glu Leu
65              70              75              80

Thr Leu Gln Ala Ser Lys Gln Asn Gly Thr Trp Pro Arg Glu Val Leu
                85              90              95

Leu Val Leu Ser Val Asn Ser Ser Val Phe Leu His Leu Gln Ala Leu
            100             105             110

Gly Ile Pro Leu His Leu Ala Tyr Asn Ser Ser Leu Val Thr Phe Gln
        115             120             125

Glu Pro Pro Gly Val Asn Thr Thr Glu Leu Pro Ser Phe Pro Lys Thr
    130             135             140

Gln Ile Leu Glu Trp Ala Ala Glu Arg Gly Pro Ile Thr Ser Ala Ala
145             150             155             160

Glu Leu Asn Asp Pro Gln Ser Ile Leu Leu Arg Leu Gly Gln Ala Gln
                165             170             175

Gly Ser Leu Ser Phe Cys Met Leu Glu Ala Ser Gln Asp Met Gly Arg
            180             185             190

Thr Leu Glu Trp Arg Pro Arg Thr Pro Ala Leu Val Arg Gly Cys His
        195             200             205

Leu Glu Gly Val Ala Gly His Lys Glu Ala His Ile Leu Arg Val Leu
    210             215             220

Pro Gly His Ser Ala Gly Pro Arg Thr Val Thr Val Lys Val Glu Leu
225             230             235             240

Ser Cys Ala Pro Gly Asp Leu Asp Ala Val Leu Ile Leu Gln Gly Pro
            245             250             255

Pro Tyr Val Ser Trp Leu Ile Asp Ala Asn His Asn Met Gln Ile Trp
            260             265             270

Thr Thr Gly Glu Tyr Ser Phe Lys Ile Phe Pro Glu Lys Asn Ile Arg
        275             280             285
```

```
Gly Phe Lys Leu Pro Asp Thr Pro Gln Gly Leu Leu Gly Glu Ala Arg
    290                 295             300

Met Leu Asn Ala Ser Ile Val Ala Ser Phe Val Glu Leu Pro Leu Ala
305             310             315                 320

Ser Ile Val Ser Leu His Ala Ser Ser Cys Gly Gly Arg Leu Gln Thr
            325             330                 335

Ser Pro Ala Pro Ile Gln Thr Thr Pro Pro Lys Asp Thr Cys Ser Pro
            340             345             350

Glu Leu Leu Met Ser Leu Ile Gln Thr Lys Cys Ala Asp Asp Ala Met
        355             360             365

Thr Leu Val Leu Lys Lys Glu Leu Val Ala His Leu Lys Cys Thr Ile
    370             375             380

Thr Gly Leu Thr Phe Trp Asp Pro Ser Cys Glu Ala Glu Asp Arg Gly
385             390             395                 400

Asp Lys Phe Val Leu Arg Ser Ala Tyr Ser Ser Cys Gly Met Gln Val
            405             410                 415

Ser Ala Ser Met Ile Ser Asn Glu Ala Val Val Asn Ile Leu Ser Ser
            420             425                 430

Ser Ser Pro Gln Arg Lys Lys Val His Cys Leu Asn Met Asp Ser Leu
        435             440             445

Ser Phe Gln Leu Gly Leu Tyr Leu Ser Pro His Phe Leu Gln Ala Ser
    450             455             460

Asn Thr Ile Glu Pro Gly Gln Gln Ser Phe Val Gln Val Arg Val Ser
465             470             475                 480

Pro Ser Val Ser Glu Phe Leu Leu Gln Leu Asp Ser Cys His Leu Asp
            485             490                 495

Leu Gly Pro Glu Gly Gly Thr Val Glu Leu Ile Gln Gly Arg Ala Ala
        500             505             510

Lys Gly Asn Cys Val Ser Leu Leu Ser Pro Ser Pro Glu Gly Asp Pro
        515             520             525

Arg Phe Ser Phe Leu Leu His Phe Tyr Thr Val Pro Ile Pro Lys Thr
    530             535             540
```

```
Gly Thr Leu Ser Cys Thr Val Ala Leu Arg Pro Lys Thr Gly Ser Gln
545             550             555             560


Asp Gln Glu Val His Arg Thr Val Phe Met Arg Leu Asn Ile Ile Ser
                565             570             575


Pro Asp Leu Ser Gly Cys Thr Ser Lys Gly Leu Val Leu Pro Ala Val
            580             585             590


Leu Gly Ile Thr Phe Gly Ala Phe Leu Ile Gly Ala Leu Leu Thr Ala
            595             600             605


Ala Leu Trp Tyr Ile Tyr Ser His Thr Arg Glu Tyr Pro Arg Pro Pro
    610             615             620


Gln
625


<210>   19
<211>   653
<212>   PRT
<213>   Mus musculus

<400>   19

Met Asp Arg Gly Val Leu Pro Leu Pro Ile Thr Leu Leu Phe Val Ile
1               5               10              15


Tyr Ser Phe Val Pro Thr Thr Gly Leu Ala Glu Arg Val Gly Cys Asp
            20              25              30


Leu Gln Pro Val Asp Pro Thr Arg Gly Glu Val Thr Phe Thr Thr Ser
            35              40              45


Gln Val Ser Glu Gly Cys Val Ala Gln Ala Ala Asn Ala Val Arg Glu
    50              55              60


Val His Val Leu Phe Leu Asp Phe Pro Gly Met Leu Ser His Leu Glu
65              70              75              80


Leu Thr Leu Gln Ala Ser Lys Gln Asn Gly Thr Glu Thr Arg Glu Val
            85              90              95


Phe Leu Val Leu Val Ser Asn Lys Asn Val Phe Val Lys Phe Gln Ala
            100             105             110


Pro Glu Ile Pro Leu His Leu Ala Tyr Asp Ser Ser Leu Val Ile Phe
            115             120             125
```

```
Gln Gly Gln Pro Arg Val Asn Ile Thr Val Leu Pro Ser Leu Thr Ser
    130             135             140

Arg Lys Gln Ile Leu Asp Trp Ala Ala Thr Lys Gly Ala Ile Thr Ser
145             150             155             160

Ile Ala Ala Leu Asp Asp Pro Gln Ser Ile Val Leu Gln Leu Gly Gln
            165             170             175

Asp Pro Lys Ala Pro Phe Leu Cys Leu Pro Glu Ala His Lys Asp Met
            180             185             190

Gly Ala Thr Leu Glu Trp Gln Pro Arg Ala Gln Thr Pro Val Gln Ser
            195             200             205

Cys Arg Leu Glu Gly Val Ser Gly His Lys Glu Ala Tyr Ile Leu Arg
    210             215             220

Ile Leu Pro Gly Ser Glu Ala Gly Pro Arg Thr Val Thr Val Met Met
225             230             235             240

Glu Leu Ser Cys Thr Ser Gly Asp Ala Ile Leu Ile Leu His Gly Pro
            245             250             255

Pro Tyr Val Ser Trp Phe Ile Asp Ile Asn His Ser Met Gln Ile Leu
            260             265             270

Thr Thr Gly Glu Tyr Ser Val Lys Ile Phe Pro Gly Ser Lys Val Lys
        275             280             285

Gly Val Glu Leu Pro Asp Thr Pro Gln Gly Leu Ile Ala Glu Ala Arg
    290             295             300

Lys Leu Asn Ala Ser Ile Val Thr Ser Phe Val Glu Leu Pro Leu Val
305             310             315             320

Ser Asn Val Ser Leu Arg Ala Ser Ser Cys Gly Gly Val Phe Gln Thr
            325             330             335

Thr Pro Ala Pro Val Val Thr Thr Pro Pro Lys Asp Thr Cys Ser Pro
            340             345             350

Val Leu Leu Met Ser Leu Ile Gln Pro Lys Cys Gly Asn Gln Val Met
            355             360             365

Thr Leu Ala Leu Asn Lys Lys His Val Gln Thr Leu Gln Cys Thr Ile
```

```
                370                      375                          380


        Thr Gly Leu Thr Phe Trp Asp Ser Ser Cys Gln Ala Glu Asp Thr Asp
        385                 390                 395                 400


        Asp His Leu Val Leu Ser Ser Ala Tyr Ser Ser Cys Gly Met Lys Val
                        405                 410                 415


        Thr Ala His Val Val Ser Asn Glu Val Ile Ile Ser Phe Pro Ser Gly
                        420                 425                 430


        Ser Pro Pro Leu Arg Lys Lys Val Gln Cys Ile Asp Met Asp Ser Leu
                435                 440                 445


        Ser Phe Gln Leu Gly Leu Tyr Leu Ser Pro His Phe Leu Gln Ala Ser
                450                 455                 460


        Asn Thr Ile Glu Leu Gly Gln Gln Ala Phe Val Gln Val Ser Val Ser
        465                 470                 475                 480


        Pro Leu Thr Ser Glu Val Thr Val Gln Leu Asp Ser Cys His Leu Asp
                        485                 490                 495


        Leu Gly Pro Glu Gly Asp Met Val Glu Leu Ile Gln Ser Arg Thr Ala
                        500                 505                 510


        Lys Gly Ser Cys Val Thr Leu Leu Ser Pro Ser Pro Glu Gly Asp Pro
                515                 520                 525


        Arg Phe Ser Phe Leu Leu Arg Val Tyr Met Val Pro Thr Pro Thr Ala
                530                 535                 540


        Gly Thr Leu Ser Cys Asn Leu Ala Leu Arg Pro Ser Thr Leu Ser Gln
        545                 550                 555                 560


        Glu Val Tyr Lys Thr Val Ser Met Arg Leu Asn Val Val Ser Pro Asp
                        565                 570                 575


        Leu Ser Gly Lys Gly Leu Val Leu Pro Ser Val Leu Gly Ile Thr Phe
                        580                 585                 590


        Gly Ala Phe Leu Ile Gly Ala Leu Leu Thr Ala Ala Leu Trp Tyr Ile
                        595                 600                 605


        Tyr Ser His Thr Arg Gly Pro Ser Lys Arg Glu Pro Val Val Ala Val
                610                 615                 620
```

```
Ala Ala Pro Ala Ser Ser Glu Ser Ser Ser Thr Asn His Ser Ile Gly
625             630             635             640


Ser Thr Gln Ser Thr Pro Cys Ser Thr Ser Ser Met Ala
            645             650
```